(19)

**Europäisches Patentamt**

**European Patent Office**

**Office européen des brevets**

(11) **EP 3 427 003 B1**

(12) **EUROPEAN PATENT SPECIFICATION**

(45) Date of publication and mention
of the grant of the patent:
**01.11.2023 Bulletin 2023/44**

(21) Application number: **17709074.3**

(22) Date of filing: **07.03.2017**

(51) International Patent Classification (IPC):
**G01B 9/02** *(2022.01)*          **G01N 21/552** *(2014.01)*
**G01N 21/45** *(2006.01)*          **G01D 5/26** *(2006.01)*
**G01N 33/68** *(2006.01)*

(52) Cooperative Patent Classification (CPC):
(C-Sets available)
**G01B 9/02051; G01B 9/02004; G01N 21/45;
G01N 21/553; G01N 33/68;** C12Q 1/6804;
C12Q 1/6816; G01N 21/554          (Cont.)

(86) International application number:
**PCT/EP2017/055269**

(87) International publication number:
**WO 2017/153378 (14.09.2017 Gazette 2017/37)**

(54) **COMPACT INTERFEROMETER, RELATED BIO-CHEMICAL SENSOR AND MEASUREMENT DEVICE**

KOMPAKTES INTERFEROMETER, ZUGEHÖRIGER BIO-CHEMISCHER SENSOR UND MESSUNGSVORRICHTUNG

INTERFÉROMÈTRE COMPACT, CAPTEUR BIOCHIMIQUE ASSOCIÉ ET DISPOSITIF DE MESURE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **08.03.2016 PCT/EP2016/054852**

(43) Date of publication of application:
**16.01.2019 Bulletin 2019/03**

(73) Proprietor: **Université de Technologie de Troyes
10000 Troyes (FR)**

(72) Inventors:
• **BRUYANT, Aurélien
10000 Troyes (FR)**
• **VAILLANT, Julien
10300 Sainte-Savine (FR)**
• **WU, Tzu-Heng
I-Lan City 26059 (TW)**
• **LIN, Chii-Wann
Taipei 106 (TW)**

(74) Representative: **Novagraaf Technologies
Bâtiment O2
2, rue Sarah Bernhardt
CS90017
92665 Asnières-sur-Seine Cedex (FR)**

(56) References cited:
**JP-A- 2007 101 242          US-A1- 2008 119 701
US-A1- 2013 329 230**

• **YASSINE HADJAR ET AL: "Compact
interferometer transducer based on surface
plasmon phase resonance", JOURNAL OF THE
OPTICAL SOCIETY OF AMERICA A, vol. 32, no.
5, 13 April 2015 (2015-04-13) , page 771,
XP55327044, US ISSN: 1084-7529, DOI:
10.1364/JOSAA.32.000771**
• **PETER KOZMA ET AL: "Integrated planar optical
waveguide interferometer biosensors: A
comparative review", BIOSENSORS AND
BIOELECTRONICS, vol. 58, 1 August 2014
(2014-08-01), pages 287-307, XP055181178, ISSN:
0956-5663, DOI: 10.1016/j.bios.2014.02.049**

**(Cont. next page)**

(52) Cooperative Patent Classification (CPC): (Cont.)

C-Sets
C12Q 1/6804, C12Q 2525/205, C12Q 2565/601;
C12Q 1/6816, C12Q 2565/601

**Description**

**TECHNICAL FIELD**

The present invention relates generally to optical measurement devices and more particularly to a compact interferometer, a related interferometric bio-chemical sensor and a corresponding measurement device.

**BACKGROUND**

Interferometry, due to its sensitivity among optical techniques, spans a wide range of applications from velocity measurement, temperature monitoring, remote sensing to bio-sensing.

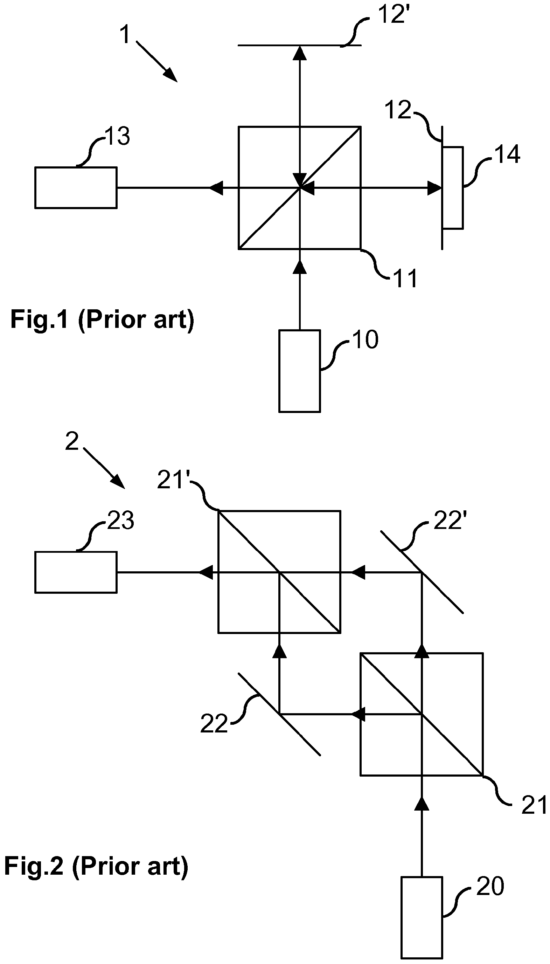

Figure 1 and Figure 2 show respectively conventional Michelson (1) and Mach-Zehnder (2) interferometers. Referring to figures 1 to 3b, in prior art, composing an interferometer requires an optical source (10, 20, 30), multiple mirrors (12, 12', 22, 22', 32) and beam splitters (11, 21, 21', 31, 31', 31"). The optical source emits a first beam toward a beam splitter that splits the first beam in two beams, respectively directed towards the two mirrors to obtain a reference beam after reflection on the first mirror and a signal beam after reflection on the second mirror. Said beam splitter or another beam splitter then recombines the reference beam and the signal beam to create an interference signal. Besides, a phase modulator (14, 34) is classically added to unambiguously discriminate the phase information from the amplitude information. When speed is not an issue, such phase modulator often consists of a piezo actuated mirror corresponding to the second mirror. Such arrangement allows the phase information carried by the signal beam light, to be recovered with respect to the phase of the reference beam light by processing the interference signal received by a photo-detector (13, 23, 33).

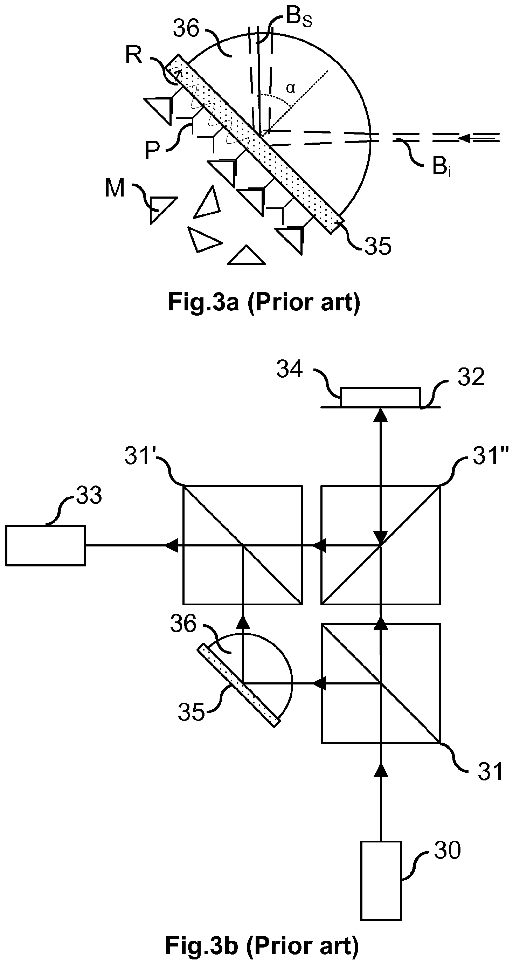

A way to confer a sensing property to an interferometer is to replace one of the mirrors by a plasmonic layer (35) coupled with a prism (36), as shown in figure 3b. Referring to figure 3a, the plasmonic layer (35) is functionalized, that is to say modified with a chemical probe (P) such as proteins, a specific sequence of deoxyribonucleic acid (DNA) or of ribonucleic acid (RNA) or other small molecules, in order to detect a specific marker (M) in a target solution. These chemical probes may directly detect a specific marker (M) or may undergo further bio-chemical reactions to detect a specific marker (M) or to amplify a specific marker (M) to enable detection.

An incident beam (Bi) is directed toward the plasmonic layer through a prism. Part of the incident beam induces an optical resonance (R) in the plasmonic layer and the other part is reflected to generate a signal beam (Bs). In the signal beam, some rays corresponding to a reflection angle $\alpha$ have undergone amplitude and phase alteration due to the optical resonance. The angle $\alpha$ is the resonance angle depending on the refractive index of the medium at the interface of the plasmonic layer. When markers are bound to the probes, the refractive index of the medium at the interface of the plasmonic layer changes and the surface plasmon resonance angle $\alpha$ is modified. This induces further amplitude and phase change of the signal beam.

Compared to its amplitude based measurement counterpart, interferometry often offers greater sensitivity and is therefore always a great candidate for sensing or measurement systems at least in calm environments. However, such interferometers are not used for large scale applications due to the need for multiple high-tech components entailing industrial and commercial drawbacks such as complex fabrication processes, bulky device, high costs mainly due to sophisticated optical sources or piezoelectric components required for phase retrieval, and technical difficulties to obtain reliable results since optical path distance variations due to mechanical vibrations are detrimental to the quality of the phase information retrieved from the light.

These drawbacks limit the possibility to develop a handheld interferometric device. However, the ever growing performances of Consumer Electronic Devices (CED) notably through smartphones, are pushing the development of smart physical sensors worldwide. In this context, it is clear to people in the field that compact interferometers are desirable to go one step further in metrological measurement system.

Weight, size and cost of sensors are the typical criteria to decide whether a sensing technology is viable. Most importantly, mechanical vibrations from environment and users are inevitable which excludes possibility for conventional interferometer to be an option in the market. Lastly, even in case of laboratory scale interferometers, a monolithic interferometer offering low noise at reasonable cost is strongly desirable.

The expanding need for portable sensors and Lab-On-Chip (LOC) system have driven a number of new solutions for compact interferometer. Examples of compact interferometric biosensors are found in the publications US 2008/119701 A1, JP 2007 101242 A, US 2013/329230 A1, Y. Hadjar er al: "Compact interferometer transducer based on surface plasmon phase resonance", JOSA A, vol. 32, no. 5 (2015), p. 771 and P. Kozma et al: "Integrated planar optical waveguide interferometer biosensors: A comparative review", Biosensors and Bioelectronics, vol. 58 (2014), p. 287-307.

Some solutions use micro-structures for wave front division, while some use micro/nano-fabrication to achieve the size reduction. However, these solutions involve fine technique in fabrication and thus high costs.

## OBJECTIVES

The present invention aims to propose interferometers, and related bio-chemical sensors and measurement device that demonstrate advantages including compactness, easy fabrication, use of low-cost optical source, and most importantly in term of performance, intrinsically low phase noise.

## SUMMARY OF EXEMPLARY EMBODIMENTS

The invention is defined in the appended claims.

Some embodiments relate to a compact and portable interferometer comprising an optical source generating a first beam; a photo-detector receiving an interference signal; an optical chip intercepting the first beam and comprising a first reflective layer and a second reflective layer separated by a dielectric spacer layer, the first reflective layer splitting the first beam into a reference beam obtained by reflection and a second beam obtained by transmission, the second reflective layer reflecting the second beam to generate a signal beam so that the signal beam overlaps the reference beam to create the interference signal; and a controlled phase modulation means inducing a phase modulation in the interference signal by modifying the phase difference between the signal beam and the reference beam, the interferometer being characterized in that the second reflective layer is an optically-resonant layer. By optically-resonant layer we mean a surface efficiently supporting an optical mode confined at the interface of the layer. This can be typically a thin metal layer supporting a surface plasmon polariton, or a localized plasmon polariton or other dielectric resonators.

By means of the optical chip that operates both beam splitting and beam recombination, the interferometer has a compact and monolithic design which brings low noise to the optical device.

In some embodiments, the interferometer further comprises a support, the optical source, the optical chip and the photo-detector being fixed onto the support. The interferometer has a packaged design which makes it easily transportable. Besides, the support enables fine-tuning of angular positioning of the optical source and the photo-detector to adapt the interferometer to reflection angles of interest.

In preferred embodiments, the dielectric spacer layer is transparent, or at least not a strongly absorbing material, with respect to the range of wavelength emission of the optical source.

In some embodiments, the controlled phase modulation means is a wavelength modulation means inducing a phase modulation in the interference signal. For example, the interferometer comprises an optical source wavelength modulation driving unit. Since the phase difference between the reference beam and the signal beam depends on the source wavelength, the wavelength modulation induces a phase modulation in the interference signal.

In other embodiments, the optical source is a truly tunable laser, for example a laser with external cavities offering a relative wavelength change close or higher than one percent, to create a phase modulation in the interferometer.

In some embodiments, the wavelength modulation means is composed of an optical source power modulation unit inducing wavelength modulation in the optical source. This power modulation method utilized to create wavelength modulation in the source avoids the need for an optical source specifically designed to create wavelength modulation in a large range. Typical low-cost solid state lasers, for example monochromatic lasers from consumer electronics like VCSEL with relative wavelength change close or smaller than $10^{-4}$%, can be sufficient to create a clear phase modulation.

In some embodiments, the dielectric spacer layer has a thickness in the range of 0.1mm to 5mm so that no delicate micro or nano-fabrication is involved which greatly facilitates the production process.

In some embodiments, the interferometer further comprises a light conveyor, such as a prism, a fiber, a grating or a lens, to convey the light to and from the bi-reflective layer at specific angles. The incident angle of the first beam is preferably adjusted with the light conveyor to induce an optical resonance in the optically-resonant layer.

Embodiments of the present invention further provide a bio-chemical sensor comprising an interferometer according to the invention wherein the optically-resonant layer has been chemically functionalized. By layer chemically functionalized, we mean a layer being modified with a chemical probe such as proteins, a specific sequence of deoxyribonucleic acid (DNA) or of ribonucleic acid (RNA) or other small molecules, in order to detect a specific marker in a target solution.

In some embodiments, the bio-chemical sensor further comprises a detection chamber attached to the optically-resonant layer and aimed to receive an analyte to be analyzed by said bio-chemical sensor. The detection chamber consists of a space that can be filled with an analyte such as a fluid. It may be a micro-fluidic channel, a gas perfusion channel, or simply an open cuvette. The detection chamber carries the target solution or gas to the optically-resonant layer surface.

In some embodiments, the optically-resonant layer comprises a plurality of distinct sub-areas arranged in a plane, different resonance properties or different functionalizations being associated to said distinctive sub-areas; and the photo-detector is arranged to receive an interference signal from each sub-area, typically using a quadrant photodiode, a linear Charged-Coupled Device (CCD), a 2D CCD, or similar detector based on Complementary Metal-Oxide Semiconductor technology (CMOS). Detecting multiple bio-markers in a diagnostic improves screening capabilities by increasing the number of test performed at once. As for traditional, amplitude based measurements, the use of a linear or 2D CCD or CMOS detector can also be used to detect light impinging on the sensing surface at different angles.

Embodiments of the present invention further provide a measurement device comprising an interferometer or a bio-chemical sensor according to the invention; and a signal analyzing unit receiving information from the photo-detector and configured to retrieve the signal beam amplitude and/or phase.

In some embodiments, the measurement device is used for monitoring Cardiac troponin I (cTnI) and the optical chip has been functionalized with a cTnI aptameric probe. The aptameric probe is comprised of specific sequences of DNA codon with one of its end being capable to bind onto the plasmonic layer for example using thiols to bind on a gold surface, or using thiol-ene click reaction or other chemical means.

In some embodiments, the measurement device is used for detection of avian influenza virus and the optical chip has been functionalized with a Hemaglutinin 5 (H5) aptamer. The aptameric probe is comprised of specific sequence of DNA codon with one of its end being capable to bind onto the plasmonic layer for example thiol gold reaction, thiol-ene click reaction or other chemical means.

In some embodiments, the measurement device is used for detection of bacteria and the optical chip has been functionalized with a specific probe.

In some embodiments, the measurement device further comprises a second photo-detector positioned in order to receive light from the first beam and sending information to the signal analyzing unit to exclude noise or amplitude modulation from the interference signal.

In some embodiments, the measurement device further comprises an additional photo-detector positioned in order to receive a portion of the interference signal, said portion being merely insensitive to the optical resonance, and sending information to the signal analyzing unit to exclude noise or amplitude modulation from the interference signal. For example, the s-polarized part of the light can be used a phase reference to correct the impact of influence quantities such as temperature.

In some embodiments, the power modulation unit and/or the signal analyzing unit are implemented by one or more portable Consumer Electronic Devices.

Additional aspects of embodiments will be set forth, in part, in the detailed description, figures and any claims which follow, and in part will be derived from the detailed description. It is to be understood that both the foregoing general description and the following detailed description are only exemplary and do not limit the claimed invention, which is defined in the appended claims.

## BRIEF DESCRIPTION OF THE DRAWINGS

Some embodiments of apparatus and/or methods will become more fully understood from the detailed description given herein below and the accompanying drawings which are given by way of illustration only and thus are not limiting of the present invention and wherein:

Figure 1 is a schematic illustration of a conventional Michelson interferometer in prior art;
Figure 2 is a schematic illustration of a conventional Mach-Zehnder interferometer in prior art;
Figure 3a schematically depicts the principle of a Surface Plasmon Resonance sensor in prior art;
Figure 3b schematically represents an interferometric Surface Plasmon Resonance sensor in prior art;
Figure 4 is a schematic view of a compact optically-resonant interferometer according to present invention;
Figure 5 is a schematic view of an interferometric bio-chemical sensor according to present invention;
Figure 6 is a schematic top view of an optically-resonant layer structured into several separated sub-area with different resonance properties and different functionalizations;
Figure 7 is a schematic view of a measurement device comprising a portable Consumer Electronic Device to process optical information;
Figure 8 is an amplitude and phase diagram generated during the binding of DNA on the optically-resonant layer using the measurement device according to the invention; and
Figure 9 shows troponin complex monitoring with a measurement device according to the invention for detection of AMI.

## DETAILED DESCRIPTION

While example embodiments are capable of various modifications and alternative forms, embodiments thereof are shown by way of example in the drawings and will herein be described in details. It should be understood, however, that there is no intent to limit example embodiments to the particular forms disclosed, but on the contrary, example embodiments are to cover all modifications, equivalents, and alternatives falling within the scope of the claims. Like numbers refer to like elements throughout the description of the figures.

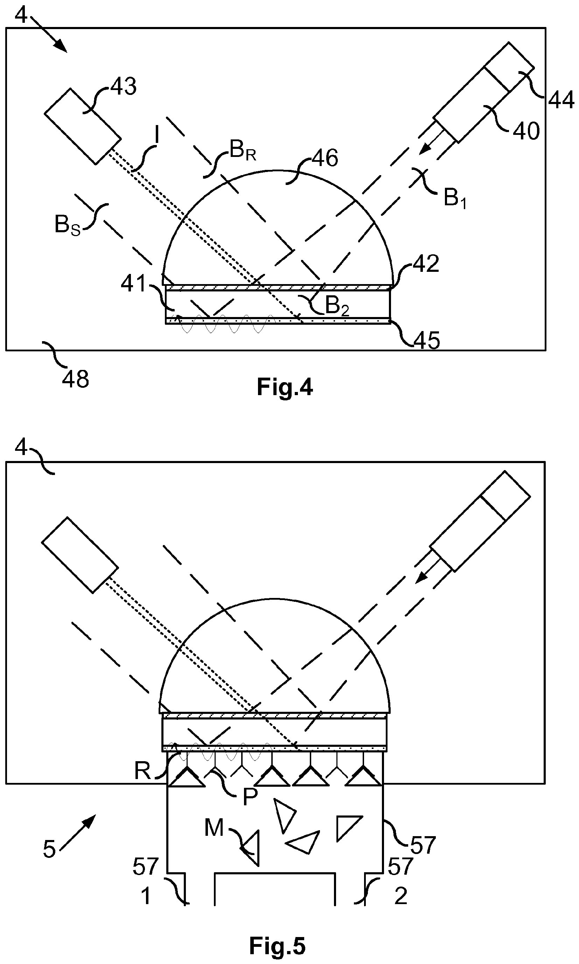

Figure 4 depicts an interferometer 4 according to the invention. The interferometer 4 comprises an optical source 40, an optical chip, a photo-detector 43 and a support 48 on which all the above components are fixed.

The optical source 40 has sufficient spectral coherence to be used in interferometry. The optical source 40 is typically a laser diode or a VCSEL. If needed, the optical source 40 is equipped with optical isolator or polarization control.

The optical chip is typically a bi-reflective layer made of isotropic and dielectric transparent materials such as optical or classical glass, plastics or organic materials. Such a bi-reflective layer offers at least two reflective surfaces at the interfaces of the layer with mediums of different refractive index. The optical chip is usually coupled with a light conveyor 46, for example a prism, a fiber, a grating or a lens, to convey the light to and from the bi-reflective layer at specific angles. The light conveyor refractive index may be the same as the one of the bi-reflective layer. In this case, no reflection would occur at the interface between the bi-reflective layer and the conveyor without depositing a thin reflective layer on this interface. In a best mode, the reflectance of the reflective surfaces is enhanced by depositing a thin reflective layer on these interfaces. On this basis, the optical chip is described as a first reflective layer 42 and a second reflective layer 45 separated by a dielectric spacer layer 41.

In a best mode, especially when the light from the optical source is diverging, the light conveyor is a half-ball lens collimating the first beam so that all the rays of the first beam are impinging near the center of the half-ball at the same incident angle on the optical chip. The use of a half-ball avoids the refraction angle produced at air-prism interface, ensuring consistent illumination condition if the angle is varied. This mode provides good measurement results even if the optical source used has poor collimation properties for example in the case of a low cost optical source.

The optical source 40 generates a first beam $B_1$ which is conveyed toward the first reflective layer 42 by a prism. The first reflective layer 42 splits the first beam $B_1$ into a reference beam $B_R$ obtained by reflection and a second beam $B_2$ obtained by transmission, the second reflective layer 45 reflects the second beam $B_2$ to generate a signal beam $B_S$ that overlaps the reference beam $B_R$ to create an interference signal I.

In order to implement phase retrieval method, the interferometer 4 comprises an optical source wavelength modulation means 44. Since the phase difference between the reference beam $B_R$ and the signal beam $B_S$ depends on the source wavelength, the wavelength modulation induces a phase modulation in the interference signal I. A first solution for wavelength modulation is to use a truly tunable laser, for example a laser with external cavities offering a relative wavelength change close or higher than one percent, as an optical source 40. Such lasers enable phase modulation in the interferometer 4. A second low cost solution for wavelength modulation is to use an optical source power modulation unit inducing a slight wavelength modulation in the optical source 40. Power modulation can be achieved by modulating the supply electric current in the optical source 40. This solution avoids the need for an optical source 40 specifically designed to create wavelength modulation in a large range. Typical low-cost solid state lasers, for example lasers from consumer electronics like VCSEL with relative wavelength change close or smaller than $10^{-4}$%, can be sufficient to create a clear phase modulation, in the order or higher than one radian, for reasonably large dielectric spacer 41, as detailed hereafter.

Unless the angle of incidence is small, the thickness of the dielectric spacer layer 41 is preferably smaller than the beam diameter coming from the optical source 40, in order to obtain sufficient overlap between the reference beam $B_R$ and the signal beam $B_S$. However, the thickness of the dielectric spacer layer 41 must be thick enough to measure a clear variation in the interference signal I due to phase modulation. The fundamental reason for having a thick enough spacer is that the phase modulation achieved by the apparatus will be proportional to this thickness and that a small modulation will result in a weak and possibly noisy signal. The thickness of the above mentioned spacer layer 41 must render phase interrogation possible with plausible range of output power modulation for optical source 40. Typically, the thickness of the above mentioned spacer layer 41 is much larger than microscale so that no tedious micro-fabrication is required. In addition, low cost optical sources that exhibit little degree of wavelength modulation are preferable. For such typical lasers working in the visible or near-infrared range, convenient spacer thicknesses in the 0.1mm to 5mm range can therefore be used, with some variations depending on the material refractive index, exact wavelength and angle of incidence. Larger wavelengths require larger thickness.

The photo-detector 43 receives the interference signal I. In this configuration, a single active area is sufficient and the photo-detector 43 is typically a single photodiode. However, a two dimensional detection device such as a CCD camera is also adapted. Based on Nyquist Sampling theorem, the photo-detector 43 has an acquisition rate at least twice times higher than the optical source wavelength modulation frequency.

The support 48 is preferably monolithic, compact and portable. The support 48 is made of rigid plastic or wood for example. Even if the support 48 is made of several elements, the interferometer can be regarded as monolithic since the heart of the interferometer, the bi-reflective optical chip, that splits and recombines the beam is a single element.

In some embodiments that are not represented, the optical source and the photo-detector positions are adjustable. For example, the support comprises two arms connected by a hinge, like a compass, in a pivot linkage with the support. The optical source and the photo-detector are placed respectively on each arm to enable fine-tuning of the angles of light emission and light reception. Said angles fine-tuning can be automatic, semi-automatic or manual.

According to the invention, the second reflective layer 45 is an optically-resonant layer. Under a predefined angle depending on the refractive index of the medium at the interface of the optically-resonant layer and on the optically-resonant layer characteristics, incident light induces an optical resonance R confined at the interface of the optically-resonant layer. The optically-resonant layer is typically a thin metal layer, usually smaller than the light wavelength in height, supporting a surface plasmon polariton. However said layer can also support a localized surface plasmon polariton (LSPP) or Dielectric Resonators (DR). LSPP or DR can increase the optical resonance and therefore increase the interferometer sensitivity to changes occurring at the layer interface. To achieve LSPP or DR, the optically resonant layer can be structured.

In figure 4, part of the second beam $B_2$ elicits an optical resonance R in the optically-resonant layer and the rest of the second beam $B_2$ is reflected toward the photo-detector 43 to generate the signal beam $B_S$.

The incident angle of the first beam B1 is preferably adjusted with the light conveyor 46 to induce an optical resonance R in the optically-resonant layer. If there is no clear optical resonant in the said layer, as for a bare dielectric surface, it is preferable to choose an angle large enough to be above the critical angle in order to still have a confined mode with an evanescent wave at the interface of the second reflective layer 45.

Figure 5 depicts a bio-chemical sensor 5 according to the invention. The bio-chemical sensor 5 comprises the interferometer 4 of figure 4 wherein the optically-resonant layer has been chemically functionalized that is to say modified with a chemical probe P such as proteins, a specific sequence of deoxyribonucleic acid (DNA) or of ribonucleic acid (RNA) or other small molecules, in order to detect a specific marker (M) in a target solution. These chemical probes may directly detect a specific marker (M) or may undergo further bio-chemical reactions to detect a specific marker (M) or to amplify a specific marker (M) to enable detection.

The bio-chemical sensor 5 further comprises a detection chamber 57 attached to the optically-resonant layer and aimed to receive an analyte. The detection chamber 57 consists of a space that can be filled with an analyte such as a fluid. It may be a micro-fluidic channel, a gas perfusion channel, or simply an open cuvette. The detection chamber 57 carries the target solution or gas to the optically-resonant layer surface. Preferably, the detection chamber 57 comprises an inlet 571 and an outlet 572.

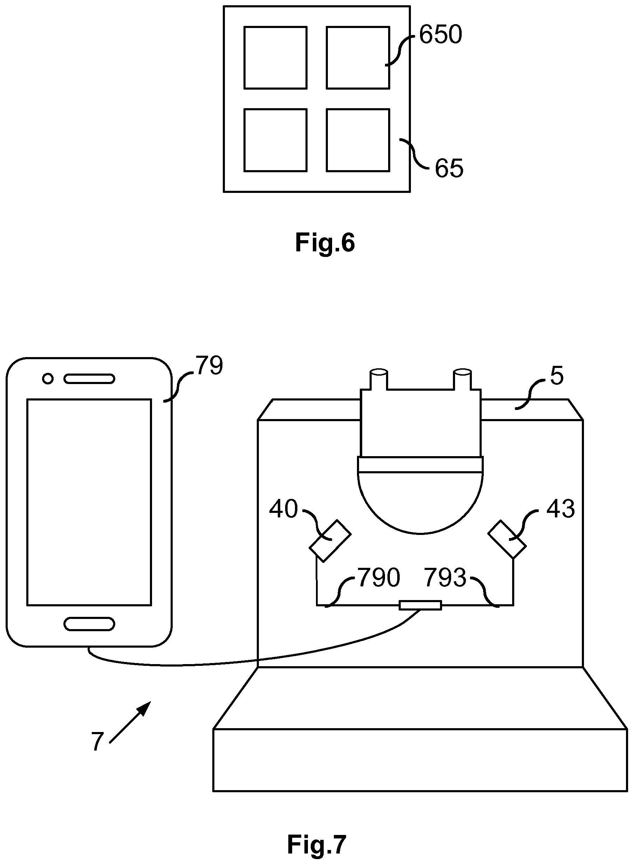

Referring to figure 6, the optically-resonant layer has been structured into several separated sub-area 650 with different resonance properties or different functionalizations in order to detect multiple bio-markers. In this example, such an optically-resonant layer is an array of plasmonic films and is referred as a plasmonic array layer 65. Each element surface in the plasmonic array layer 65 is preferably composed of the same plasmonic material with same film and resonance properties to simplify fabrication. However, the functionalization can differ from one point to the other. Nevertheless, the plasmonic array layer 65 design may be tailored to different sensing purposes. For example, the plasmonic array layer 65 design can be an array of grid, a grid being composed of nanoparticles arranged according to a pattern, with each grid having different resonance properties by use of Localized Surface Plasmon Resonance (LSPR) induced by nano-particle inside the grid. The plasmonic array layer 65 can also be a gold or silver film grid array made by one evaporation process with shadow mask. The above mentioned plasmonic array layer 65 designs are given as examples and should not be regarded as limitations, in particular regarding the grid pattern on the layer.

In figure 6 embodiment, the photo-detector 43 has to be tailored to receive an interference signal I from each sub-area 650. For example, the photo-detector 43 is a camera or a photo-detector array, that is to say an array of photo-detectors that are aligned to receive the interference signal I from each plasmonic film grid of the plasmonic array layer 65.

Figure 7 represents a measurement device 7 according to the invention. Such a measurement device 7 comprises the bio-chemical sensor 5 of figure 5 and a signal analyzing unit receiving information from the photo-detector 43 and implementing a method to retrieve the signal beam amplitude and/or phase. The signal analyzing unit as well as the power modulation unit is implemented by a smartphone 79. The smartphone is thus connected to the photo-detector 43 by a first cable 793 and is connected to the optical source 40 by a second cable 790. The signal analyzing unit and/or the power modulation unit can also be implemented by other Consumer Electronic Devices such as computers.

Actually, only few methods among which Generalized Lock-In Amplifier (G-LIA, described in the article Generalized lock-in detection for interferometry: application to phase sensitive spectroscopy and near-field nanoscopy published by OSA in Optics Express Vol. 22, Issue 18, pp. 22232-22245, also detailed in the patent applications FR301055, PCT2015036699, US2015070704) have been proposed to recover the phase information with arbitrary continuous phase modulation. Alternately, methods based on multiple harmonics detection can be used to recover the phase information in the specific case of a continuous sinusoidal phase modulation. These methods require constant amplitude during phase modulation. Unless the setup offers the possibility to normalize the amplitude, such aspect complicates the analysis and might be the reason why no similar approach has been proposed previously. To state it clearly, the specific additional difficulty here is that the phase modulation induces a simultaneous amplitude modulation which makes it difficult to distinguish the two modulation functions, amplitude and phase, since both modulate the signal at the same frequency. Even if the signal is expressed as a sum of harmonics with Fourier decomposition, it is not trivial to simply extract an optimum signal in this case, since usually a finite number of harmonics are used to recover the amplitude or phase information.

In this example, the signal analyzing unit comprises a lock-in amplifier and a signal processor to process data coming from the photo-detector in order to retrieve the amplitude and/or phase information. The lock-in amplifier works at the fundamental frequency of the modulation function and at the harmonic frequencies if present.

The invention proposes a novel approach modifying notably the G-LIA method to solve the problem. The method is expressed below in the case of a sinusoidal modulation of the reference phase, which is considered as the most convenient modulation form. A similar approach can be followed for other types of modulation function.

The optical source signal $\varepsilon_1$, that is to say the first beam signal, is expressed as follows:

$$\varepsilon_1(t) = \sqrt{2} \cdot E_1(t) \cdot \cos(\omega t + \varphi_1)$$

with $E_1$ the amplitude term, $\omega$ the angular frequency of the light and $\varphi_1$ the phase.

The optical source power P is thus:

$$P(t) \propto E_1(t)^2$$

The first beam is split into the second beam and the reference beam and the second beam is almost entirely reflected to generate the signal beam.

Using same type of notations as for $\varepsilon_1$, the reference beam signal $\varepsilon_r$ and the signal beam signal $\varepsilon_s$ are expressed as follows:

$$\varepsilon_r(t) = \sqrt{2} \cdot E_r(t) \cdot \cos(\omega t + \varphi_r)$$

$$\varepsilon_s(t) = \sqrt{2} \cdot E_s(t) \cdot \cos(\omega t + \varphi_s)$$

With these notations, the intensity I detected by the photo-detector is proportional to the temporal average of the square of the sum of the two fields since the photo-detector is in the overlapping zone:

$$I \sim \langle \left( \varepsilon_r(t) + \varepsilon_s(t) \right)^2 \rangle$$

Considering that the amplitude and phases variations are slow compared to $\omega$:

$$I \sim E_r(t)^2 + E_s(t)^2 + 2E_r(t)E_s(t)\cos(\varphi_r - \varphi_s)$$

Besides, $E_r(t)$ and $E_s(t)$ can be written as follows:

$$E_r(t) = r \cdot E_1(t)$$

$$E_s(t) = s \cdot E_1(t)$$

where $r^2 + s^2 = 1$.

Thus:

$$I \sim E_1(t)^2 \cdot (1 + 2rs \cdot \cos(\varphi_r - \varphi_s))$$

With $P(t) = E_1(t)^2$ and m=2rs:

$$I \sim P(t) \cdot (1 + m \cdot \cos(\varphi_r - \varphi_s))$$

The later relation is true for any phase modulation of the reference beam $\varphi_r(t)$. As mentioned, we consider the case of a sinusoidal phase modulation induced by a sinusoidal power modulation P(t) of the optical source:

$$P(t) = P_0(1 + \mu \sin(\Omega t))$$

And so:

$$I \sim P_0(1 + \mu \sin(\Omega t)) \cdot (1 + m \cdot \cos(\varphi_r - \varphi_s))$$

Besides, $\varphi_r$ - $\varphi_s$ depends on:

- the optical path difference L between the reference beam and the signal beam,
- the phase modification due to reflection on the first reflective layer which is constant, and
- the phase modification φ due to resonance which is the phase information that is monitored.

This phase difference Φ due to the optical path difference L can be calculated as follows:

$$\Phi = \frac{2\pi}{\lambda} \cdot L = \frac{4\pi \cdot n \cdot e \cos \theta}{\lambda}$$

with

- e: the thickness of the dielectric spacer layer,
- n: the refractive index of dielectric spacer layer,
- θ: the angle of the transmitted beam inside the dielectric spacer, with respect to the normal of the first reflective layer,
- λ: the free space wavelength of the optical source.

As power modulation induces a wavelength modulation, the wavelength λ can be written as follows:

$$\lambda(t) = \lambda_0 + \beta \sin(\Omega t)$$

Where the second term is a very small modulation compared to $\lambda_0$. Then, Φ can be written as $\Phi = \Phi_0 + d\Phi$ where $\Phi_0$ is constant and $d\Phi = -\frac{4\pi \cdot L \cdot \beta}{\lambda_0^2} \sin(\Omega t) = -a.\sin(\Omega t)$ with $a = \frac{4\pi \cdot L \cdot \beta}{\lambda_0^2}$.

Constant terms can be ignored and I can be expressed as:

$$I \sim P_0(1 + \mu \sin(\Omega t)) \cdot (1 + m \cdot \cos(a.\sin(\Omega t) - \varphi))$$

In order to retrieve the phase information φ, a first step is to apply a DC filter to I:

$$DC = \frac{1}{2\pi} \int_0^{2\pi} [P_0(1 + \mu \sin(\Omega t)) \cdot (1 + m \cdot \cos(a \cdot \sin(\Omega t) - \varphi))] \, d(\Omega t)$$

$$DC = P_0 \left( 1 + \frac{1}{2\pi} \int_0^{2\pi} [m \cdot \cos(a \cdot \sin(\Omega t) - \varphi)] \, d(\Omega t) + \frac{1}{2\pi} \int_0^{2\pi} [\mu \cdot \sin(\Omega t)] \, d(\Omega t) \right.$$

$$+ \frac{1}{2\pi} \int_0^{2\pi} \left[ \mu m \cdot \frac{1}{2} \cdot \sin(\Omega t + a \cdot \sin(\Omega t) - \varphi) \right] d(\Omega t)$$

$$\left. + \frac{1}{2\pi} \int_0^{2\pi} \left[ \mu m \cdot \frac{1}{2} \cdot \sin(\Omega t - a \cdot \sin(\Omega t) + \varphi) \right] d(\Omega t) \right)$$

Bessel functions are introduced:

$$J_n(a) = \frac{1}{2\pi} \int_0^{2\pi} [\cos(-a \cdot \sin(\Omega t) + n \cdot \Omega t)] \, d(\Omega t)$$

with following properties:

$$\frac{1}{2\pi} \int_0^{2\pi} [\cos(-a \cdot \sin(\Omega t) + n \cdot \Omega t + \varphi)] \, d(\Omega t) = \cos(\varphi) \cdot J_n(a)$$

$$\frac{1}{2\pi} \int_0^{2\pi} [\sin(-a \cdot \sin(\Omega t) + n \cdot \Omega t + \varphi)] \, d(\Omega t) = \sin(\varphi) \cdot J_n(a)$$

Thus

$$DC = P_0 + mP_0(\cos(\varphi) \cdot J_0(a) + \mu \sin(\varphi) \cdot J_1(a))$$

After applying the DC filter, $I_{\text{filter}}$ can be written as follows:

$$I_{filter} = I - DC$$

$$= P_0(m \cdot \cos(a \cdot \sin(\Omega t) - \varphi) + \mu \cdot \sin(\Omega t) + \mu m$$

$$\cdot \sin(\Omega t) \cos(a \cdot \sin(\Omega t) - \varphi) - m \cdot \cos(\varphi). J_0(a) - \mu m \cdot \sin(\varphi) \cdot J_1(a))$$

To recover amplitude and phase, an efficient approach is to perform a G-LIA on $I_{\text{filter}}$ to get:

$$R_x = \frac{1}{2\pi} \int_0^{2\pi} [I_{filter} \cdot \cos(a \cdot \sin(\Omega t))] \, d(\Omega t)$$

$$R_y = \frac{1}{2\pi} \int_0^{2\pi} [I_{filter} \cdot \sin(a \cdot \sin(\Omega t))] \, d(\Omega t)$$

After calculation, $R_x$ and $R_y$ can be expressed as:

$$R_x = \frac{P_0 m}{2} \cdot \cos(\varphi) \cdot \left( 1 + J_0(2a) - 2J_0^2(a) \right) + \frac{P_0 m \mu}{2} \cdot \sin(\varphi) \cdot \left( J_1(2a) - 2J_1(a)J_0(a) \right)$$

$$R_y = \frac{P_0 m}{2} \cdot \sin(\varphi) \cdot \left(1 - J_0(2a)\right) + \frac{P_0 m \mu}{2} \cdot \cos(\varphi) \cdot J_1(2a) + P_0 \mu J_1(a)$$

From the above expressions, amplitude m and phase $\varphi$ can be retrieved if the constants $a$ and $\mu$ are known for example via measurements.

$R_x$ and $R_y$ can be simplified by setting the phase modulation depth $a = \frac{4\pi \cdot L \cdot \beta}{\lambda_0^2} = 3.8317 rad$ and thus $J_1(a)$ = 0:

$$R_x = \frac{P_0 m}{2} \cdot \left[\cos(\varphi) \cdot \left(1 + J_0(2a) - 2J_0^2(a)\right) + \mu \cdot \sin(\varphi) \cdot J_1(2a)\right]$$

$$R_y = \frac{P_0 m}{2} \cdot \left[\sin(\varphi) \cdot \left(1 - J_0(2a)\right) + \mu \cdot \cos(\varphi) \cdot J_1(2a)\right]$$

Amplitude m and phase $\varphi$ are more easily retrieved from these simplified $R_x$ and $R_y$. In addition, simulations and experiences show that, provided $\mu \leq 0.1$, $\mu \cdot \sin(\varphi) \cdot J_1(2a)$ and $\mu \cdot \cos(\varphi) \cdot J_1(2a)$ can be neglected.
Finally, very simple expressions are obtained for $R_x$ and $R_y$:

$$R_x = \frac{P_0 m}{2} \cdot \cos(\varphi) \cdot k_x$$

$$R_y = \frac{P_0 m}{2} \cdot \sin(\varphi) \cdot k_y$$

With:

$$k_x = 1 + J_0(2a) - 2J_0^2(a) = 0.92$$

$$k_y = 1 - J_0(2a) = 0.76$$

Amplitude m and phase $\varphi$ are easily retrieved from simplified $R_x$ and $R_y$.
The original above method has been detailed for a sinusoidal power modulation function. This amplitude and phase extraction method can be performed by adequate CED such as microcontrollers, or smartphones, for example via the audio Jack plug for analog signals. Similar extraction approaches exist with a sawtooth power modulation function or other continuous wavelength modulation functions. In complex cases, calculations may involve the determination of at least one even and one odd harmonics of the interference signal I to retrieve the phase. For pure wavelength modulation, with constant or quasi constant optical source power, calculations can be simplified, and the mentioned GLIA method can be used more straightforwardly.
In some embodiments, the measurement device 7 further comprises a second photo-detector 43 positioned in order to receive light from the first beam $B_1$ and sending the information to the signal analyzing unit to exclude noise or amplitude modulation from the interference signal I.
In some embodiments, the measurement device 7 further comprises an additional photo-detector 43 positioned in order to receive a portion of the interference signal I, said portion being merely insensitive to the optical resonance modification occurring when a target is detected or more generally when the refractive index is changed. Said portion extraction is typically achieved by detecting the light polarized in a direction where the optical resonance is not perceived, known as s polarization in the case of SPR layer. Said portion can serve as a phase and amplitude reference to further reduce noises such as temperature dependence and vibration. When multiple zones are analyzed, said portion can also come from a zone insensitive to the target to serve the same purpose, for example a zone which is not functionalized. In this way, phase stability better than $10^{-4}$ rad can be achieved in the visible range.

A large number of sensing applications can be targeted by the invention, for example the measurement device can be used for medical diagnostic via the analysis of liquids such as biological fluids, including the detection of cancer gene sequence segment(s) or virial gene segment(s) with or without the use of amplification techniques such as Polymerase Chain Reaction (PCR) or Loop-mediated isothermal amplification (LAMP), detection of cancer or virus biomarkers through the use of anti-body or aptamer. Alternately, the sensing can focus on the presence of bacteria, pathogens, harmful chemicals, including gaseous molecules notably using cavitands, Heme groups or other polymeric materials as surface modification strategy. The measurement device can also be used for health prevention through bacteria detection in food for instance. The following paragraphs describe examples of possible applications.

As a first example of a possible application, the proposed invention offers a good homecare platform comprising a measurement device for patients suffering from Myocardial infarction (MI) or acute myocardial infarction (AMI), commonly known as a heart attack. Conventionally, AMI can be detected through Electro-cardiogram. However the sensitivity of the diagnostic is not good with only 57% of patients being diagnosed correctly. The measurement device monitors a proteins, Cardiac troponin I (cTnI) or troponin complex, which have been recognized as gold standard for detection of AMI.

For the measurement device to detect cTnI, the optical chip can be functionalized with a specific aptameric based probe, here a DNA sequence of around 41 mer. The handheld detection system is therefore a measurement device comprising the optical chip functionalized with the aptameric probe, the light coupling element, typically a prism, a half-cylinder, a half-ball lens or a grating in contact with the optical chip, a photo-detector, a detection chamber and signal analyzing unit receiving information from the photo-detector and configured to retrieve the signal beam phase or amplitude, the signal analyzing unit being installed on a simple micro-controller or a smartphone for example.

Blood samples are typically obtained with a lancing device and may be filtered in a buffer solution, for example with nitro-cellulose (NC) membrane filtering, before introduction into the detection chamber. For example, with the help of a lancing device, the user can obtain a tiny drop of fingertip blood. The blood then is dropped into a vial, which is prefilled with a buffer solution. The user then drops the vial into a tailor made cassette. The cassette provides a sample transfer mechanism using contact force and which transfers the content of the vial into the detection chamber for final result. After a predetermined waiting time, the endpoint data is read, and the result is transferred to a handheld device and demonstrated to user with a given user interface.

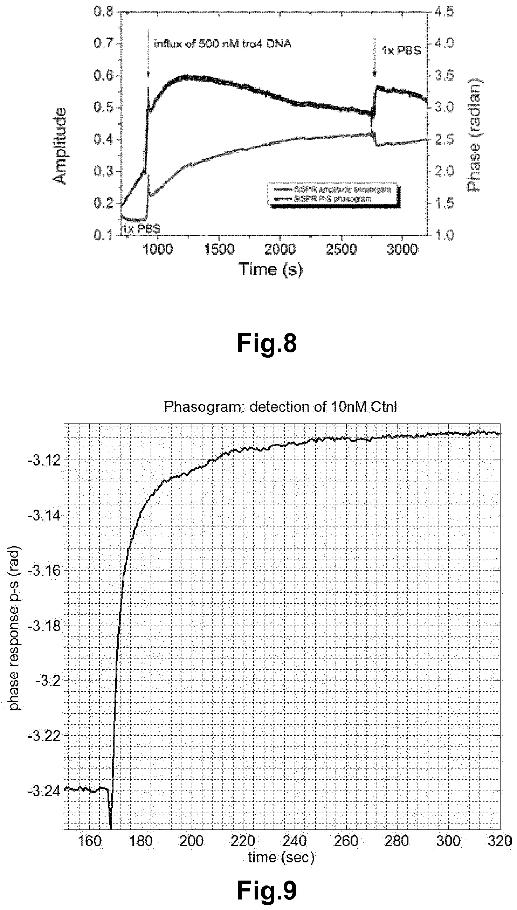

Figure 8 and Figure 9 show amplitude and phase diagrams generated by the signal analyzing unit during troponin I monitoring with a measurement device according to the invention for detection of AMI. As a first step, shown in Figure 8, the binding of the aptamer on the optical chip during the functionalization step can be monitored through DNA detection. A typical sigmoid shape is obtained corresponding to the detection of DNA. As a second step, shown in Figure 9, when the probe DNA is attached, a Phosphate-Buffered Saline (PBS) solution containing troponin complex (cTnI) with 10 nanomoles concentration is introduced into the detection chamber. A typical sigmoid shape is obtained corresponding to the detection of cTnI. From the obtained sensitivity, concentration at least one hundred times smaller can be detected in a time in the order of a few minutes or less. Targeted level of sensitivity to detect all relevant cases is 0.12 ng/mL of cTnI in the whole blood of a human being.

As a second example of a possible application, the proposed invention offers a measurement device to detect rapidly any type of virus. For example, the proposed invention offers a measurement device to detect rapidly avian influenza virus. Avian influenza has been one of the major pandemic in Asia as well as in Europe with H3N2 AIV in 2003. In 2004, H5N1 Avian influenza virus has led to about 293 deaths in Asia. AIV induces not just health care issues but also induces huge economic damages in poultry industry, notably for ducks in the south-west region in France. Typically, a poultry farm could establish several independent premises, with only thousands of poultries kept in a single premise to avoid cross infection during AIV pandemics. In the above mentioned context, the proposed cost-effective handheld device offers opportunities for on-site monitoring in poultry farms. As sensors according to the invention are affordable, series of handheld sensors can be deployed in different premises within the poultry farm. The examiner can collect urine sample from the premises for diagnostic of AIV. Monolithic sensor cassette will be used to transfer sample onto the sensor using contact force. The detection results can be collected to a computer through a network of connected sensors. In case of infection, specific premises may undergo early culling before widespread infection occurs.

To detect avian influenza, the optical chip can be functionalized with a specific aptameric based probe, here typically Hemaglutinin 5 (H5) aptamer, a 29-mer DNA sequence. The handheld detection system is therefore a measurement device comprising the optical chip functionalized with the aptameric probe, the light coupling element, typically a prism, a half-cylinder, a half-ball lens or a grating in contact with the optical chip, a photo-detector, a detection chamber and signal analyzing unit receiving information from the photo-detector and configured to retrieve the signal beam phase or amplitude, the signal analyzing unit being installed on a simple micro-controller or a smartphone for example. In this case urine of the poultry can be collected for test, typically after dilution in a PBS solution and NC membrane filtering.

As a third example of a possible application, the proposed invention offers a measurement device to detect bacteria from any source (human food, pet food, biological and physiological samples, surface wipes). In one example, the sample may come from human patients suffering from Sepsis. In case of Sepsis, the patient typically has low level of immune response due to its health issue. The reduced immune response lead to different levels of bacterial infection inside body leading to Sepsis. In another example, the sample may come from food; the detection may serve as quality control device for vegetables or other food source to exclude samples with malicious bacteria from human ingestion.

As other examples of possible applications, the measurement device can be used for detection of organic molecules, DNA or RNA, bacteria, viruses and biological markers in liquids, complex food matrices, organic matrices, food samples, tissues or blood samples, biological and physiological samples, surface wipes.

The measurement device according to the invention relies both on amplitude and on phase analysis and has therefore a better sensitivity than conventional Surface Plasmon Resonance systems relying only on amplitude analysis. Besides, the measurement device uses low cost monochromatic light sources instead of expensive spectral sources.

Compared to conventional protein analysis systems in biochemical laboratory done through the use of Enzyme-Linked ImmunoSorbent Assay (ELISA), the measurement device according to the invention has an increased sensitivity and saves a huge amount of time in sample preparation since a surface modification protocol taking several hours is needed for ELISA.

Compared to conventional Radio-Immune-Assay sensors (RIA), the measurement device according to the invention has the advantage of being suitable for on-site application.

Although some embodiments of the present invention have been illustrated in the accompanying Drawings and described in the foregoing Detailed Description, it should be understood that the present invention is not limited to the disclosed embodiments, but is capable of numerous modifications without departing from the invention as set forth and defined by the following claims.

**Claims**

1.  Interferometer (4) comprising:

    - an optical source (40) configured to generate a first beam ($B_1$);
    - a photo-detector (43) configured to receive an interference signal (I);
    - a monolithic optical chip configured to intercept the first beam ($B_1$), that is a bi-reflective layer offering at least two reflective surfaces at the interfaces of the bi-reflective layer with mediums of different refractive index and comprising a first top reflective layer (42) and a second bottom reflective layer (45) separated by an intermediary isotropic and dielectric spacer layer (41), the first top reflective layer (42) splitting the first beam ($B_1$) into a reference beam ($B_R$) obtained by reflection and a second beam ($B_2$) obtained by transmission, the second bottom reflective layer (45) being an optically-resonant layer reflecting the second beam ($B_2$) to generate a signal beam ($B_S$) so that the signal beam ($B_S$) overlaps the reference beam ($B_R$) to create the interference signal (I); **characterized in that** it further comprises a controlled phase modulation means configured to induce a phase modulation in the interference signal (I) by modifying the phase difference between the signal beam (Bs) and the reference beam ($B_R$).

2.  Interferometer (4) according to claim 1 further comprising a support (48), the optical source (40), the optical chip and the photo-detector (43) being fixed onto the support (48).

3.  Interferometer (4) according to claim 1 or 2 wherein the controlled phase modulation means is a wavelength modulation means (44) inducing a phase modulation in the interference signal (I).

4.  Interferometer (4) according to claim 3 wherein the wavelength modulation means (44) is composed of an optical source power modulation unit inducing wavelength modulation in the optical source (40).

5.  Interferometer (4) according to any one of claims 1 to 4 further comprising a light conveyor (46) to convey the light to and from the bi-reflective layer at specific angles.

6.  Bio-chemical sensor (5) **characterized in that** it comprises an interferometer (4) according to any one of claims 1 to 5 wherein the second bottom optically-resonant layer (45) has been chemically functionalized.

7.  Bio-chemical sensor (5) according to claim 6 further comprising a detection chamber (57) attached to the lower surface of the second bottom optically-resonant layer and aimed to receive an analyte to be analyzed by said bio-chemical sensor.

8.  Bio-chemical sensor (5) according to claim 6 or 7 wherein:

- the optically-resonant second bottom layer (45) comprises a plurality of distinct sub-areas (650), different resonance properties or different functionalizations being associated to said distinct sub-areas (650); and
- the photo-detector (43) is arranged to receive an interference signal (I) from each sub-area (650).

9. Measurement device (7) **characterized in that** it comprises:

   - an interferometer (4) according to any one of claims 1 to 5 or a bio-chemical sensor (5) according to any one of claims 6 to 8; and
   - a signal analyzing unit receiving information from the photo-detector (43) and configured to retrieve the signal beam ($B_S$) amplitude and/or phase.

10. Measurement device (7) according to claim 9 for monitoring Cardiac troponin I (cTnI) wherein the optical chip has been functionalized with a cTnI aptameric probe.

11. Measurement device (7) according to claim 9 for detection of avian influenza virus wherein the optical chip has been functionalized with a Hemaglutinin 5 (H5) aptamer.

12. Measurement device (7) according to claim 9 for detection of bacteria wherein the optical chip has been functionalized with a specific probe.

**Patentansprüche**

1. Interferometer (4), umfassend:

   - eine optische Quelle (40), die konfiguriert ist, um einen ersten Strahl ($B_1$) zu erzeugen;
   - einen Photodetektor (43), der konfiguriert ist, um ein Störsignal (I)zu empfangen;
   - einen monolithischen optischen Chip, der konfiguriert ist, um den ersten Strahl ($B_1$) abzufangen, d. h. eine bi-reflektierende Schicht, die mindestens zwei reflektierende Oberflächen an den Schnittstellen der bi-reflektierenden Schicht mit Medien von unterschiedlichem Brechungsindex bietet und umfassend eine erste obere reflektierende Schicht (42) und eine zweite untere reflektierende Schicht (45), die durch eine dazwischenliegende isotrope und dielektrische Abstandshalterschicht (41) getrennt sind, wobei die erste obere reflektierende Schicht (42) den ersten Strahl ($B_1$) in einen Referenzstrahl ($B_R$) trennt, der durch Reflexion erhalten wird, und einen zweiten Strahl ($B_2$), der durch Übertragung erhalten wird, wobei die zweite untere reflektierende Schicht (45) eine optisch resonante Schicht ist, die den zweiten Strahl ($B_2$) reflektiert, um eine Signalstrahl ($B_S$) zu erzeugen, so dass der Signalstrahl ($B_S$) den Referenzstrahl ($B_R$) überlagert, um das Interferenzsignal (I) zu erschaffen; **dadurch gekennzeichnet, dass** es ferner ein gesteuertes Phasenmodulationsmittel umfasst, das konfiguriert ist, um eine Phasenmodulation in dem Interferenzsignal (I) durch Modifizieren der Phasendifferenz zwischen dem Signalstrahl ($B_S$) und dem Referenzstrahl ($B_R$) zu induzieren.

2. Interferometer (4) nach Anspruch 1, ferner umfassend einen Träger (48), wobei die optische Quelle (40), der optische Chip und der Photodetektor (43) auf dem Träger (48) befestigt sind.

3. Interferometer (4) nach Anspruch 1 oder 2, wobei das gesteuerte Phasenmodulationsmittel ein Wellenlängenmodulationsmittel (44) ist, das eine Phasenmodulation in dem Interferenzsignal (I) induziert.

4. Interferometer (4) nach Anspruch 3, wobei das Wellenlängenmodulationsmittel (44) aus einer optischen Quellen-Leistungsmodulationseinheit zusammengesetzt ist, die eine Wellenlängenmodulation in der optischen Quelle (40) induziert.

5. Interferometer (4) nach einem der Ansprüche 1 bis 4, ferner umfassend einen Lichtförderer (46), um das Licht zu und von der bi-reflektierenden Schicht in spezifischen Winkeln zu befördern.

6. Biochemischer Sensor (5), **dadurch gekennzeichnet, dass** er ein Interferometer (4) nach einem der Ansprüche 1 bis 5 umfasst, wobei die zweite untere optisch resonante Schicht (45) chemisch funktionalisiert wurde.

7. Biochemischer Sensor (5) nach Anspruch 6, ferner umfassend eine Detektionskammer (57), die an der unteren Oberfläche der zweiten unteren optisch resonanten Schicht angebracht und darauf gerichtet ist, einen Analyten zu empfangen, der durch den biochemischen Sensor analysiert werden soll.

8. Biochemischer Sensor (5) nach Anspruch 6 oder 7, wobei:

   - die optisch-resonante zweite untere Schicht (45) eine Vielzahl von eindeutigen Unterbereichen (650) umfasst, wobei unterschiedliche Resonanzeigenschaften oder unterschiedliche Funktionalisierungen den verschiedenen Unterbereichen (650) zugeordnet sind; und
   - der Photodetektor (43) angeordnet ist, um ein Störsignal (I) von jedem Unterbereich (650) zu empfangen.

9. Messvorrichtung (7), **dadurch gekennzeichnet, dass** sie umfasst:

   - ein Interferometer (4) nach einem der Ansprüche 1 bis 5 oder einen biochemischen Sensor (5) nach einem der Ansprüche 6 bis 8; und
   - eine Signalanalyseeinheit, die Informationen von dem Photodetektor (43) empfängt und konfiguriert ist, um die Signalstrahl ($B_S$) -Amplitude und/oder -Phase abzurufen.

10. Messvorrichtung (7) nach Anspruch 9 zum Überwachen von Herztroponin I (cTnI), wobei der optische Chip mit einer cTnI-Aptamer-Sonde funktionalisiert wurde.

11. Messvorrichtung (7) nach Anspruch 9 für die Detektion des Vogel-Influenzavirus, wobei der optische Chip mit einem Hämagluinin 5 (H5) -Aptamer funktionalisiert wurde.

12. Messvorrichtung (7) nach Anspruch 9 für die Detektion von Bakterien, wobei der optische Chip mit einer spezifischen Sonde funktionalisiert wurde.


**Revendications**

1. Interféromètre (4) comprenant :

   - une source optique (40) configurée pour générer un premier faisceau (B1) ;
   - un photo-détecteur (43) configuré pour recevoir un signal d'interférence (I) ;
   - une puce optique monolithique configurée pour intercepter le premier faisceau ($B_1$), laquelle est une couche bi-réfléchissante offrant au moins deux surfaces réfléchissantes aux interfaces de la couche bi-réfléchissante avec des milieux d'indice de réfraction différent et comprenant une première couche réfléchissante supérieure (42) et une deuxième couche réfléchissante inférieure (45) séparées par une couche intermédiaire d'espacement isotrope et diélectrique (41), la première couche réfléchissante supérieure (42) divisant le premier faisceau ($B_1$) en un faisceau de référence ($B_R$) obtenu par réflexion et un second faisceau ($B_2$) obtenu par transmission, la deuxième couche réfléchissante inférieure (45) étant une couche optiquement résonante réfléchissant le second faisceau ($B_2$) pour générer un faisceau de signal ($B_S$) de sorte que le faisceau de signal ($B_S$) se superpose avec le faisceau de référence ($B_R$) pour créer le signal d'interférence (I) ; **caractérisé en ce qu'**il comprend en outre un moyen de modulation de phase contrôlé configuré pour induire une modulation de phase dans le signal d'interférence (I) en modifiant la différence de phase entre le faisceau de signal ($B_S$) et le faisceau de référence ($B_R$).

2. Interféromètre (4) selon la revendication 1 comprenant en outre un support (48), la source optique (40), la puce optique et le photo-détecteur (43) étant fixés sur le support (48).

3. Interféromètre (4) selon la revendication 1 ou 2, dans lequel le moyen de modulation de phase contrôlé est un moyen de modulation de longueur d'onde (44) induisant une modulation de phase dans le signal d'interférence (I).

4. Interféromètre (4) selon la revendication 3, dans lequel le moyen de modulation de longueur d'onde (44) est composé d'une unité de modulation de puissance de la source optique induisant une modulation de longueur d'onde dans la source optique (40).

5. Interféromètre (4) selon l'une des revendications 1 à 4, comprenant en outre un guide de lumière (46) pour acheminer la lumière vers et depuis la couche bi-réfléchissante à des angles spécifiques.

**6.** Capteur biochimique (5) **caractérisé en ce qu'**il comprend un interféromètre (4) selon l'une quelconque des revendications 1 à 5, dans lequel la deuxième couche inférieure optiquement résonante (45) a été chimiquement fonctionnalisée.

**7.** Capteur biochimique (5) selon la revendication 6, comprenant en outre une chambre de détection (57) fixée à la surface inférieure de la deuxième couche inférieure optiquement résonnante et destinée à recevoir un analyte devant être analysé par ledit capteur biochimique.

**8.** Capteur biochimique (5) selon la revendication 6 ou 7 dans lequel :

- la deuxième couche inférieure optiquement résonante (45) comprend une pluralité de sous-zones distinctes (650), différentes propriétés de résonance ou différentes fonctionnalisations étant associées à ces sous-zones distinctes (650) ; et
- le photo-détecteur (43) est conçu pour recevoir un signal d'interférence (I) de chaque sous-zone (650).

**9.** Dispositif de mesure (7) **caractérisé en ce qu'**il comprend :

- un interféromètre (4) selon l'une des revendications 1 à 5 ou un capteur biochimique (5) selon l'une des revendications 6 à 8 ; et
- une unité d'analyse des signaux recevant des informations du photo-détecteur (43) et configurée pour récupérer l'amplitude et/ou la phase du faisceau de signal (Bs).

**10.** Dispositif de mesure (7) selon la revendication 9 pour le contrôle de la troponine cardiaque I (cTnI) dans lequel la puce optique a été fonctionnalisée avec une sonde aptamérique cTnI.

**11.** Dispositif de mesure (7) selon la revendication 9 pour la détection du virus de la grippe aviaire, dans lequel la puce optique a été fonctionnalisée avec un aptamère de l'hémaglutinine 5 (H5).

**12.** Dispositif de mesure (7) selon la revendication 9 pour la détection des bactéries, dans lequel la puce optique a été fonctionnalisée avec une sonde spécifique.

**Fig.1 (Prior art)**

**Fig.2 (Prior art)**

**Fig.3a (Prior art)**

**Fig.3b (Prior art)**

**Fig.4**

**Fig.5**

**Fig.6**

**Fig.7**

**Fig.8**

**Fig.9**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 2008119701 A1
- JP 2007101242 A
- US 2013329230 A1
- FR 301055
- FR 2015036699 W
- US 2015070704 A

### Non-patent literature cited in the description

- **Y. HADJAR.** Compact interferometer transducer based on surface plasmon phase resonance. *JOSA A,* 2015, vol. 32 (5), 771
- **P. KOZMA et al.** Integrated planar optical waveguide interferometer biosensors: A comparative review. *Biosensors and Bioelectronics,* 2014, vol. 58, 287-307
- Generalized lock-in detection for interferometry: application to phase sensitive spectroscopy and near-field nanoscopy. Optics Express. OSA, vol. 22, 22232-22245